# EUROPEAN PATENT SPECIFICATION

(11) **EP 1 631 383 B1**
(45) Date of publication and mention of the grant of the patent: **18.07.2007**
(21) Application number: 04753512.5
(22) Date of filing: 27.05.2004
(51) Int. Cl.: B01J 23/34, B01J 23/75, C07C 51/265, C07C 57/34

(54) **OXIDATION OF AROMATIC HYDROCARBONS USING BROMINATED ANTHRACENE PROMOTERS**
OXIDATION VON AROMATISCHEN KOHLENWASSERSTOFFEN UNTER VERWENDUNG VON BROMIERTEN ANTHRACENEN ALS PROMOTOREN
OXYDATION D'HYDROCARBURES AROMATIQUES AU MOYEN DE PROMOTEURS A L'ANTHRACENE BROME

(30) Priority: 06.06.2003 US 476687 P
(43) Date of publication of application: 08.03.2006
(73) Proprietor: BP Corporation North America Inc., Warrenville, Illinois 60555 (US)
(72) Inventor: METELSKI, Peter, D., Bolingbrook, IL 60490 (US); ESPENSON, James, H., Ames, IA 50014 (US)
(74) Representative: Hamer, Christopher K.
(86) International application number: PCT/US2004/016694
(87) International publication number: WO 2005/000779

(56) References cited:
- EP-A- 0 315 100
- EP-A- 0 578 369
- WO-A-82/03625

## Description

The U.S. Government has rights in this invention pursuant to Agreement No. AL-WFO-97-01.

### Background of the Invention

Aromatic carboxylic acids such as benzene dicarboxylic acids and naphthalene dicarboxylic acids are commercially valuable as the raw materials for manufacture of polyester materials which are used to manufacture fibers, films, resins, and many other petrochemical compounds. U.S. Patent No. 2,833,816, discloses the liquid phase oxidation of xylene isomers into corresponding benzene dicarboxylic acids in the presence of bromine using a catalyst having cobalt and manganese components. As described in WO82/03625 and U.S. Patent No. 5,103,933, liquid phase oxidation of dimethyl naphthalenes to naphthalene dicarboxylic acids can also be accomplished in the presence of bromine and a catalyst having cobalt and manganese components. Typically, aromatic carboxylic acids are purified in a subsequent process as described, for example, in U.S. Patent No. 3,584,039, U.S. Patent No. 4,892,972, and U.S. Patent No. 5,362,908.

The liquid phase oxidation of aromatic hydrocarbons to aromatic carboxylic acid is conducted using a reaction mixture comprising aromatic hydrocarbons and a solvent. Typically, the solvent comprises a C₁-C₈ monocarboxylic acid, for example acetic acid, benzoic acid, or mixtures thereof with water. As used herein, "aromatic hydrocarbon" preferably means a molecule composed predominantly of carbon atoms and hydrogen atoms, and having one or more aromatic ring, particularly dimethyl benzenes and dimethyl naphthalenes. Aromatic hydrocarbons suitable for liquid-phase oxidation to produce aromatic carboxylic acid generally comprise an aromatic hydrocarbon having at least one substituent group that is oxidizable to a carboxylic acid group. As used herein, "aromatic carboxylic acid" preferably means an aromatic hydrocarbon with at least one carboxyl group.

A bromine promoter and catalyst are added to the reaction mixture which is reacted in the presence of an oxidant gas. Typically, the catalyst comprises at least one suitable heavy metal component. Suitable heavy metals include heavy metals with atomic weight in the range of about 23 to about 178. Examples include cobalt, manganese, vanadium, molybdenum, chromium, iron, nickel, zirconium, cerium or a lanthanide metal such as hafnium. Suitable forms of these metals include for example, acetates, hydroxides, and carbonates.

The use of bromine in producing aromatic carboxylic acids by liquid phase oxidation improves conversion of the reactants, however, bromine also presents some drawbacks. For example, a portion of the bromine in the reaction mixture reacts with alkyl groups to produce alkyl bromide gas, for example methyl bromide, an undesirable gas which necessitates costly treatment and disposal. Additionally, bromine and its byproducts contribute to corrosion of the oxidation reaction vessel and equipment used to process the reaction products. Therefore, the reactor and process equipment must be designed to withstand the corrosive effects, generally by using specialty stainless steels or titanium metallurgy.

The corrosion compounds which develop as a result of the interaction between bromine and process equipment can contaminate the aromatic carboxylic acid product. The aromatic carboxylic acid product is typically subjected to a subsequent purification process. The purification process may include treating the aromatic carboxylic acid product with hydrogen gas in the presence of a hydrogenation catalyst. Corrosion compounds present in the aromatic carboxylic acid product are detrimental to the hydrogenation catalyst.

Quality of aromatic carboxylic acids is often determined by the concentration of intermediate products found as impurities in the aromatic carboxylic acid product. The type and concentration of these impurities varies with the types and concentrations of catalyst and promoter used and with the particular aromatic carboxylic acid product desired. The presence of such impurities may interfere with use of the carboxylic acid product. For example, when terephthalic acid is used in a direct condensation process in preparing polyesters, impurities in the terephthalic acid can cause undesirable coloration of the polyester and can act as chain terminators.

All impurities in aromatic carboxylic acid products have not been identified, however, one or more identified impurity is often used as an indicator of the purity of an aromatic carboxylic acid product. For example, 4-carboxybenzaldehyde ("4-CBA") is an intermediate oxidation product formed during oxidation of paraxylene in a process for making terephthalic acid. It is known that coloration induced in polyesters formed from terephthalic acid can be correlated with the 4-CBA concentration in the terephthalic acid. 4-CBA does not necessarily promote coloration during polymerization, however, it can be used as a tracer for evaluating the degree to which color inducing impurities are present in terephthalic acid.

Many modifications and improvements have been made to the liquid-phase oxidation process, for example: U.S. Patent No. 6,194,607 to Jhung et al. discloses the addition of an alkali metal or alkaline earth metal to the reaction mixture in the oxidation of xylene isomers to benzene dicarboxylic acids; U.S. Patent 5112,992 to Belmonte et al. discloses the addition of hafnium to oxidation catalysts; U.S. Patent No. 5,081,290 to Partenheimer et al. discloses manipulation of acetate concentration to control the rate of oxidation. None of these disclosures directly address the detrimental corrosive effects of bromine nor do they directly address the formation of alkyl bromide.

Attempts have been made to reduce the amount of bromine present in the oxidation reaction to reduce its detrimental effects. However, reduction in the molar ratio of bromine to catalyst components can cause an unacceptable precipitation of catalyst components. Such precipitation may result in discoloration of the aromatic carboxylic acid product which is not desirable. In commercial scale operations precipitation of catalyst components hinders process flow and product recovery.

There is a need in the art for a significant reduction in the corrosion and contamination attributable to bromine without significant reduction in conversion rate and product quality. Additionally, there is a need to reduce the production of alkyl bromide gas, particularly methyl bromide, without significant reduction in product yield or quality.

We have discovered that brominated anthracenes, either alone or in combination with conventional bromine sources, are effective as a promoter for the liquid-phase oxidation of aromatic hydrocarbons. The liquid-phase oxidation of aromatic hydrocarbons to form aromatic carboxylic acids can be carried out in the presence of a promoter comprising brominated anthracene, preferably a mono- or dibromoanthracene, more preferably 9-bromoanthracene or 9,10-dibromoanthracene, and a metal catalyst, preferably comprising cobalt and either manganese, cerium, or both, resulting in yields and conversions comparable to the reaction using a conventional bromine promoter. As used herein, "brominated anthracene" means an anthracene molecule with one or more bromine atoms substituted for one or more hydrogen atoms such that the position of the bromine does not inhibit its function as a promoter of the oxidation reaction.

Use of brominated anthracene as a bromine promoter in the oxidation reaction also reduces the amount of alkyl bromide produced in the reaction thereby reducing the cost of treatment and handling of waste gases. Corrosion of the reactor and process equipment is also reduced and the resulting product contamination is reduced.

Another difficulty encountered in the liquid phase oxidation of aromatic hydrocarbons to form aromatic carboxylic acids is solvent and aromatic hydrocarbon burning. The liquid phase oxidation reaction typically results in the burning of at least 2% of the solvent and more than 2% of the aromatic hydrocarbon. We have discovered that the use of brominated anthracene as a promoter can reduce one or both of solvent burning and hydrocarbon burning.

### Summary of the Invention

This invention provides a catalyst system for liquid-phase oxidation of aromatic hydrocarbons to form aromatic carboxylic acid at a temperature in the range from about 120°C to about 250°C. The catalyst system comprises at least one suitable heavy metal and one or more brominated anthracene. Optionally, the catalyst system can further comprise a conventional bromine source which preferably is one or more bromine compounds selected from the group consisting of Br₂, HBr, NaBr, KBr, NH₄Br, benzyl-bromide, bromo acetic acid, dibromo acetic acid, tetrabromoethane, ethylene dibromide and bromoacetyl bromide. Preferably the heavy metal and brominated anthracene are present in a solvent comprising a C₁-C₈ monocarboxylic acid. The heavy metal preferably comprises cobalt and one or more secondary metals selected from the group consisting of manganese, cerium, zirconium and hafnium, and is preferably present in an amount ranging from 100 ppmw to 6,000 ppmw. Preferably, the atom ratio of elemental bromine to heavy metal ranges from 0.1:1 to 4:1. The brominated anthracene preferably comprises 9-bromoanthracene or 9,10-dibromoanthracene.

This invention also provides a process for oxidizing aromatic hydrocarbons with an oxidant gas to aromatic carboxylic acids in a reaction solvent comprising a C₁-C₈ monocarboxylic acid under liquid phase conditions at temperatures in the range from 120°C to 250°C. The process comprises oxidizing aromatic hydrocarbons in the presence of a catalyst comprising at least one suitable heavy metal and one or more brominated anthracene. Preferably, the heavy metal comprises cobalt and one or more secondary metals selected from the group consisting of manganese, cerium, zirconium, and hafnium. The heavy metal preferably is present in an amount ranging from 100 ppmw to 6000 ppmw. Preferably, oxidation is conducted at a pressure in the range from 5 to 40 kg/cm² gauge. The aromatic hydrocarbons preferably consist essentially of paraxylene. The brominated anthracene preferably comprises 9-bromoanthracene or 9,10-dibromoanthracene.

This invention also provides a process for reducing the formation of alkyl bromide during the production of aromatic carboxylic acids by oxidizing aromatic hydrocarbons in a reaction solvent comprising a C₁-C₈ monocarboxylic acid. The process comprises adding a catalyst to the reaction solvent wherein the catalyst comprises at least one suitable heavy metal, adding a bromine promoter to the reaction solvent wherein the bromine promoter comprises one or more brominated anthracenes, and performing the oxidation at a temperature in the range of 120 °C to 250 °C. Optionally, the bromine promoter can further comprise one or more bromine compounds selected from the group consisting of Br₂, HBr, NaBr, KBr, NH₄Br, benzyl-bromide, bromo acetic acid, dibromo acetic acid, tetrabromoethane, ethylene dibromide and bromoacetyl bromide. Preferably, the heavy metal comprises cobalt and one or more secondary metals selected from the group consisting of manganese, cerium, zirconium, and hafnium. The heavy metal preferably is present in an amount ranging from 100 ppmw to 6000 ppmw. The aromatic hydrocarbons preferably consist essentially of paraxylene. Preferably, the oxidation is conducted at a pressure in the range of 5 to 40 kg/cm² gauge. The brominated anthracene preferably comprises 9-bromoanthracene or 9,10-dibromoanthracene.

### Description of the Preferred Embodiment(s)

The liquid-phase oxidation of aromatic hydrocarbons to produce aromatic carboxylic acids can be conducted as a batch process, a continuous process, or a semicontinuous process. The oxidation reaction can be conducted in one or more reactors. A reaction mixture is formed by combining an aromatic hydrocarbon feed, solvent, catalyst, and a bromine promoter. In a continuous or semicontinuous process, the reaction mixture components preferably are combined in a mixing vessel before being introduced into the oxidation reactor, however, the reaction mixture can be formed in the oxidation reactor.

Aromatic carboxylic acids for which the invention is suited include mono- and polycarboxylated species having one or more aromatic rings and which can be manufactured by reaction of gaseous and liquid reactants in a liquid phase system, and especially those in which solid reaction products are produced and/or liquid components of the reaction mixture enter a vapor phase above the liquid phase in the reactor. Examples of aromatic carboxylic acids for which the invention is particularly suited include trimesic acid, trimellitic acid, phthalic acid, isophthalic acid, terephthalic acid, benzoic acid and naphthalene dicarboxylic acids.

Suitable aromatic hydrocarbon feed generally comprises an aromatic hydrocarbon having at least one group that is oxidizable to a carboxylic acid group. The oxidizable substituent or substituents can be an alkyl group, such as a methyl, ethyl or isopropyl group. It also can be a group already containing oxygen, such as a hydroxyalkyl, formyl or keto group. The substituents can be the same or different. The aromatic portion of feedstock compounds can be a benzene nucleus or it can be bi- or polycyclic, such as a naphthalene nucleus. The number of oxidizable substituents on the aromatic portion of the feedstock compound can be equal to the number of sites available on the aromatic portion, but is generally fewer than all such sites, preferably 1 to about 4 and more preferably 1 to 3. Examples of useful feed compounds include toluene, ethylbenzene, o-xylene, p-xylene, m-xylene, 1-formyl-4-methylbenzene, 1-hydroxymethyl-4-methylbenzene, 1,2,4-trimethylbenzene, 1-formyl-2,4-dimethylbenzene, 1,2,4,5-tetramethylbenzene, alkyl-, hydroxymethyl-, formyl- and acyl-substituted naphthalene compounds, such as 2,6- and 2,7-dimethylnaphthalenes, 2-acyl-6-methylnaphthalene, 2-formyl-6-methylnaphthalene, 2-methyl-6-ethylnaphthalene and 2,6-diethylnaphthalene.

For manufacture of aromatic carboxylic acids by oxidation of corresponding aromatic hydrocarbon pre-cursors, e.g., manufacture of isophthalic acid from meta-disubstituted benzenes, terephthalic acid from para-disubstituted benzenes, trimellitic acid from 1,2,4-trisubstituted benzenes, naphthalene dicarboxylic acids from disubstituted naphthalenes, it is preferred to use relatively pure feed materials, and more preferably, feed materials in which content of the pre-cursor corresponding to the desired acid is at least about 95 wt.%, and more preferably at least 98 wt.% or even higher. A preferred aromatic hydrocarbon feed for use to manufacture terephthalic acid comprises paraxylene. A preferred feed for isophthalic acid comprises metaxylene. A preferred feed for trimellitic acid comprises 1,2,4-trimethylbenzene. Toluene is a preferred feed material for making benzoic acid.

Solvents comprising an aqueous carboxylic acid, and especially a lower alkyl (e.g., C₁-C₈) monocarboxylic acid, for example acetic or benzoic acid, are preferred because they tend to be only sparingly prone to oxidation under typical oxidation reaction conditions used for manufacture of aromatic acids, and can enhance catalytic effects in the oxidation. Specific examples of suitable carboxylic acids include acetic acid, propionic acid, butyric acid, benzoic acid and mixtures thereof. Ethanol and other co-solvent materials which oxidize to monocarboxylic acids under the aromatic acid oxidation reaction conditions also can be used as is or in combination with carboxylic acids with good results. Of course, for purposes of overall process efficiency and minimizing separations, it is preferred that when using a solvent comprising a mixture of monocarboxylic acid and such a co-solvent, the co-solvent should be oxidizable to the monocarboxylic acid with which it is used.

Catalysts used according to the invention comprise materials that are effective to catalyze oxidation of the aromatic hydrocarbon feed to aromatic carboxylic acid. Preferably, the catalyst is soluble in the liquid oxidation reaction body to promote contact among catalyst, oxygen and liquid feed; however, heterogeneous catalyst or catalyst components may also be used. Typically, the catalyst comprises at least one suitable heavy metal component such as a metal with atomic weight in the range of 23 to 178. Examples of suitable heavy metals include cobalt, manganese, vanadium, molybdenum, chromium, iron, nickel, zirconium, cerium or a lanthanide metal such as hafnium. Suitable forms of these metals include for example, acetates, hydroxides, and carbonates. The catalyst of this invention preferably comprises cobalt compounds alone or in combination with one or more of manganese compounds, cerium compounds, zirconium compounds, or hafnium compounds.

A bromine promoter is used to promote oxidation activity of the catalyst metal, preferably without generation of undesirable types or levels of by-products, and is preferably used in a form that is soluble in the liquid reaction mixture. The bromine promoter of this invention comprises one or more brominated anthracenes. Brominated anthracenes are commercially available from bulk chemical vendors such as TCI Chemicals, Sigma-Aldrich Bulk Division and R. W. Greef. It is typically available as a solid at 95% to 99% purity. The brominated anthracenes can be used in combination with conventional bromine promoters. Brominated anthracenes in combination with conventional bromine promoters can result in slightly better conversion and less expense but also may increase corrosion and alkyl bromide production when compared to brominated anthracene promoters alone. Preferably, the bromine promoter comprises mono-bromoanthracene, dibromoanthracene or both, more preferably the bromine promoter comprises 9-bromoanthracene, 9,10-dibromoanthracene or both. Conventional bromine promoters include Br₂, HBr, NaBr, KBr, NH₄Br, benzyl-bromide, bromo acetic acid, dibromo acetic acid, tetrabromoethane, ethylene dibromide and bromoacetyl bromide. The bromine in conventional bromine promoters liberates to a greater extent than the bromine in brominated anthracene promoters. Liberated bromine reacts with process equipment and alkyl groups resulting in corrosion, contamination, and alkyl bromide gas. It has been found that extent of reaction of alkyl groups with bromine in brominated anthracene is significantly less than with bromine from conventional bromine promoters.

Use of other brominated aromatic compounds as bromine promoters, such as bromobenzenes, bromonaphthalenes, bromopyrenes, bromoteracenes, bromochrysenes, bromotriphenylenes, was investigated. Surprisingly, it was found that brominated anthracenes are significantly better bromine promoters than other brominated aromatic compounds. Brominated anthracene promoters resulted in product yields and quality comparable to conventional bromine promoters, however, other brominated aromatic promoters resulted in significantly lower product yield, higher burning, or both in comparison to conventional bromine promoters.

The oxidation reaction is conducted in an oxidation reactor. The oxidation reactor can comprise one or more reactor vessels. Oxidant gas is also introduced into the oxidation reactor. Oxidant gas used according to the invention comprises molecular oxygen. Air is conveniently used as a source of molecular oxygen. Oxygen-enriched air, pure oxygen and other gaseous mixtures comprising at least about 10% molecular oxygen also are useful. As will be appreciated, as molecular oxygen content of the source increases, compressor requirements and handling of inert gases in reactor off-gases are reduced.

Proportions of the feed, catalyst, oxygen and solvent are not critical to the invention and vary not only with choice of feed materials and intended product but also choice of process equipment and operating factors. Solvent to feed weight ratios suitably range from 1:1 to 10:1. Oxidant gas typically is used in at least a stoichiometric amount based on feed but not so great that unreacted oxygen escaping from the liquid body to the overhead gas phase forms a flammable mixture with other components of the gas phase. Catalysts suitably are used in concentrations of catalyst metal, based weight of aromatic hydrocarbon feed and solvent, greater than 100 ppmw, preferably greater than 500 ppmw, and less than 10,000 ppmw, preferably less than 6,000 ppmw, more preferably less than 3000 ppmw. Bromine promoter preferably is present in an amount such that the atom ratio of bromine to catalyst metal suitably is greater than 0.1:1, preferably greater than 0.2:1 and suitably is less than 4:1, preferably less than 3:1. In accordance with this invention the promoter comprises one or more brominated anthracene, either alone or in combination with conventional bromine promoters, in an amount such that the atom ratio of bromine to catalyst metal most preferably ranges from 0.25:1 to 2:1. In a preferred embodiment of this invention, one or more brominated anthracene provides at least 50% of the atomic bromine in the oxidation reaction.

Oxidation of aromatic hydrocarbon feed materials to product comprising aromatic carboxylic acid is conducted under oxidation reaction conditions. The reaction is operated at temperatures sufficient to drive the oxidation reaction at a commercially reasonable rate, and provide desirable purity. Heat generated by oxidation is dissipated to maintain reaction conditions. Typically, heat of reaction is dissipated by boiling and condensing the reaction mixture. Generally suitable temperatures are in excess of 120°C, preferably in excess of 150°C, and less than 250°C preferably less than 230°C. At temperatures lower than 120°C the oxidation reaction proceeds too slowly and results in insufficient product purity. For example, oxidation of paraxylene to produce terephthalic acid at a temperature less than 120 °C takes more than 24 hours to proceed to substantial completion. The resultant terephthalic acid product requires significant additional processing due to its high level of impurities. The substantial heat generated when oxidizing commercial quantities of paraxylene at temperatures below 120 °C can not be dissipated effectively.

Pressure in the reaction vessel is at least high enough to maintain a substantial liquid phase comprising feed and solvent in the vessel. Generally, pressures of 5 to 40 kg/cm² gauge are suitable, with preferred pressures for particular processes varying with feed and solvent compositions, temperatures and other factors. Solvent residence times in the reaction vessel can be varied as appropriate for given throughputs and conditions, with 20 to 150 minutes being generally suited to a range of processes. For processes in which the aromatic acid product is substantially soluble in the reaction solvent, such as in the manufacture of trimellitic acid by oxidation of psuedocumene in acetic acid solvent, solid concentrations in the liquid body are negligible. In other processes, such as oxidation of xylenes to isophthalic or terephthalic acids, solids contents can be as high as 50 wt.% of the liquid reaction body, with levels of about 10 to 35 wt.% being more typical. As will be appreciated by those skilled in the manufacture of aromatic acids, preferred conditions and operating parameters vary with different products and processes and can vary within or even beyond the ranges specified above.

In a particular embodiment, the invention is used for the boiling liquid phase oxidation of an aromatic hydrocarbon feed comprising paraxylene to terephthalic acid. Liquid components comprising the aromatic hydrocarbon feed and solvent are continuously introduced into the reaction vessel. Catalyst and promoter, each most preferably also dissolved in solvent, are introduced into the vessel.

Acetic acid or aqueous acetic acid is a preferred solvent, with a solvent to feed ratio of 2:1 to 5:1 being preferred. The catalyst preferably comprises cobalt in combination with manganese, cerium, zirconium, hafnium, or any combination thereof. A mono- or dibromoanthracene, or a combination thereof is preferably used as promoter, more preferably 9-bromoanthracene, 9,10-dibromoanthracene, or a combination thereof is used as promoter. The catalyst is suitably present in amounts providing 600 ppmw to 2500 ppmw of catalyst metals based on weight of the aromatic hydrocarbon and solvent. The promoter most preferably is present in an amount such that the atom ratio of bromine to catalyst metal is 0.4:1 to 1.5:1.

Oxidant gas is supplied to one or more reactors at a rate effective to provide at least about 3 moles molecular oxygen per mole of aromatic hydrocarbon feed material, and such that unreacted oxygen in the vapor space above the liquid reaction body is below the flammable limit. When air is used as an oxidant gas, the limit is about 8 mole % when measured after removal of condensable compounds.

The reaction vessel is preferably maintained at 150 to 225°C under pressure of 5 to 40 kg/cm² gauge. Under such conditions, contact of the oxygen and feed material in the liquid body results in formation of solid terephthalic acid crystals, typically in finely divided form. Solids content of the boiling liquid slurry typically ranges up to 40 wt.% and preferably from 20 to 35 wt.%, and water content typically is 5 to 20 wt.% based on solvent weight. Boiling of the liquid body for control of the reaction exotherm causes volatilizable components of the liquid body, including solvent and water of reaction, to vaporize within the liquid. Unreacted oxygen and vaporized liquid components escape from the liquid into the reactor space above the liquid. Other species, for example nitrogen and other inert gases that are present if air is used as an oxidant gas, carbon oxides, and vaporized byproducts, e.g., methyl acetate and methyl bromide, also may be present in the overhead vapor, however this invention reduces the amount of methyl bromide formed.

The overhead vapor typically is removed from the reaction vessel and can be subjected to further processing such as separation and condensation of solvent vapors and water for recycle to the reactor, removal of water for disposal or use in other process steps, treatment to remove gaseous by-products, and energy recovery.

Aromatic carboxylic acid reaction product, slurried or dissolved in a portion of the liquid body, is removed from the vessel. The product stream can be treated using conventional techniques to separate its components and to recover the aromatic carboxylic acid contained therein, usually by crystallization, liquid-solid separations and drying. Conveniently, a slurry of solid product in the liquid is centrifuged, filtered or both, in one or more stages. Soluble product dissolved in the liquid can be recovered by crystallization. Liquid comprising water, solvent, unreacted feed material, and often also containing one or more liquid catalyst, promoter and reaction intermediates, can be returned to the reaction vessel.

Aromatic carboxylic acid product recovered from the liquid can be used or stored as is, or it may be subjected to purification or other processing. Purification is beneficial for removing by-products and impurities that may be present with the aromatic carboxylic acid that is recovered. For aromatic carboxylic acids such as terephthalic and isophthalic acids, purifications preferably involves hydrogenation of the oxidation product, typically dissolved in water or other aqueous solvent, at elevated temperature and pressure in the presence of a catalyst comprising a metal with hydrogenation catalytic activity, such as ruthenium, rhodium, platinum or palladium, which typically is supported on carbon, titania or other suitable, chemically-resistant supports or carriers for the catalyst metal. Purification processes are known, for example, from US 3,584,039, US 4,782,181, 4,626,598 and US 4,892,972. If purification is conducted with water as solvent, washing with water to remove residual oxidation solvent from the solid aromatic carboxylic acid can be carried out as an alternative to drying. Such washing can be accomplished using suitable solvent exchange devices, such as filters, as disclosed in US 5,679,846, US 5,175,355 and US 5,200,557.

Typically, mother liquor is separated from the aromatic carboxylic acid product through separation techniques known in the art, for example, filtration, centrifuge, or combinations of known methods. It is preferable to recycle at least a portion of the mother liquor and commercial operations typically recycle a significant portion of the mother liquor.

Conversion rates for liquid phase oxidation of aromatic hydrocarbon feedstock to aromatic carboxylic acid using brominated anthracene promoters were observed to be comparable to conversion rates using conventional promoters such as HBr or NaBr. One of the benefits of this invention is that the reaction conversion rate is not significantly affected by substituting brominated anthracene for conventional bromine promoters.

Another advantage of this invention is that use of a brominated anthracene promoter in liquid phase oxidation reactions liberates less bromine than conventional bromine promoters. By decreasing the liberated bromine, brominated anthracene promoters reduce corrosion of process equipment and reduce the formation of alkyl bromide gas.

The invention is further described in the following examples, which are presented for purposes of illustration, not limitation.

### COMPARATIVE EXAMPLE A

Liquid phase oxidation of paraxylene to form terephthalic acid ("TA") was conducted without a bromine promoter for comparative purposes. The reactor used was a 71 ml titanium vessel equipped with a thermocouple and a valve for adding gas. During the reaction, the vessel was agitated using mechanical shaking. Heating was provided by mechanically raising a fluidized sand bath to immerse the agitated reactor into thermostatted sand. The reactor temperature was controlled by adjusting the height of the sand bath relative to the reactor to maintain the temperature indicated by the internal thermocouple. At the completion of the heating cycle, rapid cooling was accomplished by lowering the fluidized sand bath and directing water jets onto the exterior surface of the reactor. The off-gas was vented into a gas sampling bag for analysis and the solid and liquid contents of the reactor were recovered and analyzed.

The reactor was charged with a solution of Mn(OAc)2·4H2O (0.0270 g), Co(OAc)₂·4H₂O (0.0265 g), HBr (0.0087 g), and 9.5 g of 95% (by volume) aqueous acetic acid. Before sealing, 0.50 g paraxylene was added. The reactor was sealed and pressurized to 35 kg/cm² (gauge) with air. The reactor was heated to 180 °C and held at this temperature with agitation for 10 minutes. At the end of this time, the reactor was removed from the heat and cooled quickly to room temperature using a water spray. The contents of the reactor were analyzed. The TA yield was measured and an indication of product quality was determined by the concentration of 4-CBA. Burning ratio was determined as a molar fraction of carbon oxides in the off-gas to paraxylene feed. Concentration of methyl bromide in the off-gas was also measured. The results are reported in Table 1.

### EXAMPLE 1

A stock solution was prepared in a 50 ml volumetric flask by adding to the flask 0.52g Co(OAc)₂·4H₂O, 0.065g Mn(OAc)₂·4H₂O, and filling the flask to the 50 ml mark with 95% (aqueous) acetic acid. A reactor identical to the reactor used in Comparative Example A was charged with 9.5 g stock solution, 0.5 g paraxylene, and 0.03 g 9,10-dibromoanthracene solid acquired from Sigma-Aldrich Bulk Division. The reactor was pressurized to 35 kg/cm² (gauge) with air and sealed. The reaction ran for 10 minutes at 170 °C after which time the reactor was rapidly cooled to room temperature using a water spray. The contents of the reactor were analyzed. The TA yield was measured and an indication of product quality was determined by the concentration of 4-CBA. Burning ratio was determined as a molar fraction of carbon oxides in the off-gas to paraxylene feed. Concentration of methyl bromide in the off-gas was also measured. The results are reported in Table 1.

### EXAMPLE 2

The reaction was conducted as in Example 1, except that in place of 9,10-dibromoantracene, 0.0095 g of 9-bromoanthracene solid acquired from Sigma-Aldrich Bulk Division was added to the reactor. The results are reported in Table 1.

### EXAMPLE 3

A stock solution was prepared in a 50 ml volumetric flask by adding to the flask 0.52g Co(OAc)₂·4H₂O, 0.09g Ce(OAc)₃·1½H₂O, and filling the flask to the 50 ml mark with 95% (aqueous) acetic acid. A reactor identical to the reactor used in Comparative Example A was charged with 9.5 g stock solution, 0.5 g paraxylene, and 0.02 g 9,10-dibromoanthracene. The reactor was pressurized to 35 kg/cm² (gauge) with air and sealed. The reaction ran for 10 minutes at 165 °C after which time the reactor was rapidly cooled to room temperature using a water spray. The contents of the reactor were analyzed. The TA yield was measured and an indication of product quality was determined by the concentration of 4-CBA. Burning ratio was determined as a molar fraction of carbon oxides in the off-gas to paraxylene feed. Concentration of methyl bromide in the off-gas was also measured. The results are reported in Table 1.

### EXAMPLE 4

The reaction was conducted as in Example 1, except that in place of 9,10-dibromoantracene, 0.13 g of 9-bromoanthracene was added to the reactor and the reaction was conducted at a temperature of 170 °C. Results are reported in Table 1.

| TABLE 1 | | | | | | | | | | |
|---|---|---|---|---|---|---|---|---|---|---|
| Example No. | [Co] mM | [Mn] mM | [Ce] mM | [DBA] mM | [9BA] mM | [HBr] mM | TA % Yield | 4-CBA % | [MeBr] ppmv | Burning Ratio |
| A | 36 | 1 | 0 | 0 | 0 | 12 | 98.3 | 1.4 | 16 | 0.46 |
| 1 | 40 | 5 | 0 | 10 | 0 | 0 | 97.5 | 1.9 | 15 | 0.34 |
| 2 | 40 | 5 | 0 | 0 | 5 | 0 | 96.3 | 2.8 | 4 | 0.36 |
| 3 | 40 | 0 | 5 | 6.2 | 0 | 0 | 98.2 | 1.1 | 12 | 0.27 |
| 4 | 40 | 0 | 5 | 0 | 5 | 0 | 97.3 | 2.1 | 6.7 | 0.42 |

The reactions conducted using a brominated anthracene promoter resulted in quality and yield comparable to the reaction conducted using a hydrogen bromide promoter. Additionally, use of a brominated anthracene promoter produced less methyl bromide gas and resulted in less solvent burning. The results in Table 1 demonstrate the successful use of brominated anthracenes to promote the catalytic oxidation of an aromatic hydrocarbon to form an aromatic carboxylic acid.

## Claims

1. A catalyst system for producing aromatic carboxylic acid by liquid-phase oxidation of aromatic hydrocarbons at a temperature in the range from about 120°C to about 250°C, the catalyst system comprising:
a) at least one suitable heavy metal; and
b) at least one brominated anthracene.

2. The catalyst system of Claim 1 further comprising a conventional bromine source.

3. The catalyst system of Claim 2 wherein the conventional bromine source comprises one or more bromine compound selected from the group consisting of Br₂, HBr, NaBr, KBr, NH₄Br, benzyl-bromide, bromo acetic acid, dibromo acetic acid, tetrabromoethane, ethylene dibromide and bromoacetyl bromide

4. The catalyst system of Claim 1 wherein the at least on suitable heavy metal and the at least one brominated anthracene are present in a solvent comprising a C₁-C₈ monocarboxylic acid.

5. The catalyst system of Claim 1 wherein the at least one suitable heavy metal comprises cobalt and one or more secondary metals selected from the group consisting of manganese, cerium, zirconium and hafnium.

6. The catalyst system of Claim 5 wherein the at least one suitable heavy metal is present in an amount ranging from 100 ppmw to 6000 ppmw.

7. The catalyst system of Claim 1 wherein the atom ratio of elemental bromine to at least one suitable heavy metal ranges from 0.1:1 to 4:1.

8. The catalyst system of Claim 1 wherein the brominated anthracene comprises 9-bromoanthracene.

9. The catalyst system of Claim 1 wherein the brominated anthracene comprises 9,10-dibromoanthracene.

10. A process for oxidizing aromatic hydrocarbons with an oxidant gas to form aromatic carboxylic acids in a reaction solvent comprising a C₁-C₈ monocarboxylic acid under liquid phase conditions at temperatures in the range from 120°C to 250°C, the process comprising oxidizing aromatic hydrocarbons in the presence of catalyst comprising at least one suitable heavy metal and one or more brominated anthracene.

11. The process of Claim 10 wherein the at least one suitable heavy metal comprises cobalt and one or more .secondary metal selected from the group consisting of manganese, cerium, zirconium and hafnium.

12. The process of Claim 11 wherein the at least one suitable heavy metal is present in an amount ranging from 100 ppmw to 6000 ppmw.

13. The process of Claim 10 wherein oxidation is conducted at a pressure in the range from 5 to 40 *kglcm²* gauge.

14. The process of Claim 13 wherein the aromatic hydrocarbons consist essentially of paraxylene.

15. The process of Claim 10 wherein the brominated anthracene comprises 9-bromoanthracene.

16. The process of Claim 10 wherein the brominated anthracene comprises 9,10-dibromoanthracene.

17. A process for reducing the formation of alkyl bromide during the production of aromatic carboxylic acid by oxidizing aromatic hydrocarbons in a reaction solvent comprising a C₁-C₈ monocarboxylic acid, the process comprising:
a) adding a catalyst to the reaction solvent wherein the catalyst comprises at least one suitable heavy metal;
b) adding a bromine promoter to the reaction solvent wherein the bromine promoter comprises one or more brominated anthracenes; and
c) oxidizing aromatic hydrocarbons at a temperature in the range of 120 °C to 250 °C.

18. The process of Claim 17 wherein the bromine promoter further comprises one or more bromine compounds selected from the group consisting of Br₂, HBr, NaBr, KBr, NH₄Br, benzyl-bromide, bromo acetic acid, dibromo acetic acid, tetrabromoethane, ethylene dibromide and bromoacetyl bromide.

19. The process of Claim 17 wherein the at least one suitable heavy metal comprises cobalt and one or more secondary metals selected from the group consisting of manganese, cerium, zirconium and hafnium.

20. The process of Claim 19 wherein the at least one suitable heavy metal is present in an amount ranging from 100 ppmw to 6000 ppmw.

21. The process of Claim 17 wherein the brominated anthracene comprises 9-bromoanthracene.

22. The process of Claim 17 wherein the brominated anthracene comprises 9,10-dibromoanthracene.

23. The process of Claim 17 wherein the aromatic hydrocarbons consist essentially of paraxylene.

24. The process of Claim 23 wherein the step of oxidizing aromatic hydrocarbons at a temperature in the range of 120 °C to 250 °C is performed at a pressure in the range from 5 to 40 kg/cm² gauge.

## Patentansprüche

1. Katalysatorsystem zur Erzeugung von aromatischer Carbonsäure durch Flüssigphasenoxidation von aromatischen Kohlenwasserstoffen bei einer Temperatur im Bereich von etwa 120°C bis etwa 250°C, wobei das Katalysatorsystem enthält:
a) wenigstens ein geeignetes Schwermetall;
b) wenigestens ein bromiertes Anthracen.

2. Katalysatorsystem nach Anspruch 1, weiterhin enthaltend einen üblichen Brom-Ausgangsstoff.

3. Katalysatorsystem nach Anspruch 2, wobei der übliche Brom-Ausgangsstoff eine oder mehrere Verbindungen aus der Gruppe: Br₂, HBr, NaBr, KBr, NH₄Br, Benzylbromid, Bromessigsäure, Dibromessigsäure, Tetrabromethan, Ethylendibromid und Bromacetylbromid enthält.

4. Katalysatorsystem nach Anspruch 1, worin das wenigstens eine geeignete Schwermetall und das wenigstens eine bromierte Anthracen in einer Lösung vorliegen, die eine C₁-C₈-Monokarbonsäure enthält.

5. Katalysatorsystem nach Anspruch 1, wobei das wenigstens eine geeignete Schwermetall Kobalt und wenigstens ein oder mehrere sekundäre Metalle aus der Gruppe Mangan, Cer, Zirkonium und Hafnium enthält.

6. Katalysatorsystem nach Anspruch 5, wobei das wenigstens eine geeignete Schwermetall in einer Menge im Bereich ovn 100 ppmw bis 6000 ppmw vorliegt.

7. Katalysatorsystem nach Anspruch 1, wobei das Atomverhältnis von elementarem Brom zu wenigstens einem geeigneten Schwermetall im Bereich von 0,1:1 bis 4:1 liegt.

8. Katalaysatorsystem nach Anspruch 1, wobei das bromierte Anthracen 9-Bromoanthracen enthält.

9. Katalysatorsystem nach Anspruch 1, wobei das bromierte Anthracen 9,10-Dibromoanthracen enthält.

10. Verfahren zum Oxidieren von armomatischen Kohlenwasserstoffen mit einem oxidierenden Gas zur Bildung aromatischer Karbonsäuren in einem Reaktionslösungsmittel, das eine C₁-C₈-Monokarbonsäure enthält, unter Flüssigphasenbedingungen bei Temperaturen im Bereich von 120°Cbis 250°C, wobei das Verfahren Oxidierung von aromatischen Kohlenwasserstoffen in Gegenwart eines Katalysators, der wenigstens ein geeignetes Schwermetall und ein oder mehrere bromierte Athracene enthält, umfasst.

11. Verfahren nach Anspruch 10, wobei das wenigstens eine geeignete Schmermetall Kobalt und wenigstens ein oder mehrere Sekundärmetall(e) aus der Gruppe Kobalt und Mangan, Cer, Zirkonium und Hafnium enthält.

12. Verfahren nach Anspruch 11, wobei das wenigstens eine geeignete Schwermetall in einer Menge im Bereich von 100 ppmw bis 6000 ppmw vorliegt.

13. Verfahren nach Anspruch 10, wobei die Oxidation bei einem Druck im Bereich von 5 bis 40 kg/cm² Überdruck durchgeführt wird.

14. Verfahren nach Anspruch 13, wobei die aromatischen Kohlenwasserstoffe im Wesentlichen aus Paraxylol bestehen.

15. Verfahren nach Anspruch 10, wobei das bromierte Anthracen 9-Bromoanthracen enthält.

16. Verfahren nach Anspruch 10, wobei das bromierte Anthracen 9,10-Dibromoanthracen enthält.

17. Verfahren zur Verringerung der Bildung von Alkylbromid während der Herstellung von aromatischer Karbonsäure durch Oxidierung von aromatischen Kohlenwasserstoffen in einem Reaktionslösungsmittel, das eine C₁-C₈-Monokarbonsäure enthält, umfassend:
a) die Zugabe eines Katalysators zum Reaktionslösungsmittel, wobei der Katalysator wenigstens ein geeignetes Schwermetall enthält;
b) die Zugabe eines Brom-Promotors zu dem Reaktionslösungsmittel, wobei der Brom-Promotor ein oder mehrere bromierte(s) Anthracen(e) enthält; und
c) Oxidierung von aromatischen Kohlenwasserstoffen bei einer Temperatur im Bereich von 120°C bis 250°C.

18. Verfahren nach Anspruch 17, wobei der Brom-Promotor weiterhin ein oder mehrere Brom-Verbindungen aus der Gruppe: Br₂, HBr, NaBr, KBr, NH₄Br, Benzylbromid, Bromessigsäure, Dibromessigsäure, Tetrabromethan, Ethylendibromid und Bromacetylbromid enthält.

19. Verfahren nach Anspruch 17, wobei das wenigstens eine geeignete Schwermetall Kobalt und ein oder mehrere sekundäre(s) Metall(e) aus der Gruppe: Mangan, Cer, Zirkonium und Hafnium enthält.

20. Verfahren nach Anspruch 19, wobei das wenigstens eine geeignete Schwermetall in einer Menge im Bereich von 100 ppmw bis 6000 ppmw vorliegt.

21. Verfahren nach Anspruch 17, wobei das bromierte Anthracen 9-Bromoanthracen enthält.

22. Verfahren nach Anspruch 17, wobei das bromierte Anthracen 9,10-Dibromoanthracen enthält.

23. Verfahren nach Anspruch 17, wobei die aromatischen Kohlenwasserstoffe im Wesentlichen aus Paraxylol bestehen.

24. Verfahren nach Anspruch 23, wobei der Schritt der Oxidierung aromatischer Kohlenwasserstoffe bei einer Temperatur im Bereich von 120°C bis 250°C bei einem Druck im Bereich von 5 bis 40 kg/cm² Überdruck ausgeführt wird.

## Revendications

1. Système catalyseur destiné à produire un acide carboxylique aromatique par oxydation en phase liquide d'hydrocarbures aromatiques à une température dans la plage d'environ 120°C à environ 250°C, le système catalyseur comprenant :
a) au moins un métal lourd approprié ; et
b) au moins un anthracène bromé.

2. Système catalyseur selon la revendication 1 comprenant en outre une source conventionnelle de brome.

3. Système catalyseur selon la revendication 2, dans lequel la source conventionnelle de brome comprend un ou plusieurs composés bromés choisis dans le groupe constitué de Br₂, HBr, NaBr, KBr, NH₄Br, bromure de benzyle, acide bromo acétique, acide dibromo acétique, tétrabromoéthane, dibromure d'éthylène et bromure de bromoacétyle.

4. Système catalyseur selon la revendication 1, dans lequel l'au moins un métal lourd approprié et l'au moins un anthracène bromé sont présents dans un solvant comprenant un acide monocarboxylique en C₁ à C₈.

5. Système catalyseur selon la revendication 1, dans lequel l'au moins un métal lourd approprié comprend le cobalt et un ou plusieurs métaux secondaires choisis dans le groupe constitué du manganèse, du cérium, du zirconium et du hafnium.

6. Système catalyseur selon la revendication 5, dans lequel l'au moins un métal lourd approprié est présent en une quantité allant de 100 ppm en poids à 6 000 ppm en poids.

7. Système catalyseur selon la revendication 1, dans lequel le rapport atomique du brome élémentaire sur au moins un métal lourd approprié s'étend de 0,1 : 1 à 4 : 1.

8. Système catalyseur selon la revendication 1, dans lequel l'anthracène bromé comprend le 9-bromoanthracène.

9. Système catalyseur selon la revendication 1, dans lequel l'anthracène bromé comprend le 9,10-dibromoantracène.

10. Procédé destiné à oxyder des hydrocarbures aromatiques par un gaz oxydant pour former des acides carboxyliques aromatiques dans un solvant de réaction comprenant un acide monocarboxylique en C₁ à C₈ dans des conditions de phase liquide à des températures dans la plage de 120°C à 250°C, le procédé comprenant des hydrocarbures aromatiques oxydants en présence d'un catalyseur comprenant au moins un métal lourd approprié et un ou plusieurs anthracènes bromés.

11. Procédé selon la revendication 10, dans lequel l'au moins un métal lourd approprié comprend le cobalt et un ou plusieurs métaux secondaires sont choisis dans le groupe constitué du manganèse, du cérium, du zirconium et du hafnium.

12. Procédé selon la revendication 11, dans lequel l'au moins un métal lourd approprié est présent en une quantité allant de 100 ppm en poids à 6 000 ppm en poids.

13. Procédé selon la revendication 10, dans lequel l'oxydation est conduite sous une pression dans la plage de 5 à 40 kg/cm² au manomètre.

14. Procédé selon la revendication 13, dans lequel les hydrocarbures aromatiques sont constitués essentiellement de paraxylène.

15. Procédé selon la revendication 10, dans lequel l'anthracène bromé comprend le 9-bromoanthracène.

16. Procédé selon la revendication 10, dans lequel l'anthracène bromé comprend le 9,10-dibromoanthracène.

17. Procédé destiné à réduire la formation de bromure d'alkyle pendant la production de l'acide carboxylique aromatique en oxydant les hydrocarbures aromatiques dans un solvant de réaction comprenant un acide monocarboxylique en C₁ à C₈, le procédé comprenant les étapes suivantes :
a) ajouter un catalyseur au solvant de réaction, dans laquelle le catalyseur comprend au moins un métal lourd approprié ;
b) ajouter un promoteur bromé au solvant de réaction, dans laquelle le promoteur bromé comprend un ou plusieurs anthracènes bromés ; et
c) oxyder les hydrocarbures aromatiques à une température dans la plage de 120°C à 250°C.

18. Procédé selon la revendication 17, dans lequel le promoteur bromé comprend en outre un ou plusieurs composés bromés choisis dans le groupe constitué de Br₂, HBr, NaBr, KBr, NH₄Br, bromure de benzyle, acide bromo acétique, acide dibromo acétique, tétrabromoéthane, dibromure d'éthylène et bromure de bromoacétyle.

19. Procédé selon la revendication 17, dans lequel l'au moins un métal lourd approprié comrend le cobalt et un ou plusieurs métaux secondaires sont choisis dans le groupe constitué du manganèse, du cérium, du zirconium et du hafnium.

20. Procédé selon la revendication 19, dans lequel l'au moins un métal lourd approprié est présent en une quantité allant de 100 ppm en poids à 6 000 ppm en poids.

21. Procédé selon la revendication 17, dans lequel l'anthracène bromé comprend le 9-bromoanthracène.

22. Procédé selon la revendication 17, dans lequel l'anthracène bromé comprend le 9,10-dibromoanthracène.

23. Procédé selon la revendication 17, dans lequel les hydrocarbures aromatiques sont constitués essentiellement de paraxylène.

24. Procédé selon la revendication 23, dans lequel l'étape d'oxydation des hydrocarbures aromatiques à une température dans la plage de 120°C à 250°C est effectuée à une pression dans la plage de 5 à 40 kg/cm² au manomètre.
